# EUROPEAN PATENT APPLICATION

(11) **EP 4 851 733 A1**
(43) Date of publication of application: **09.12.2026**
(21) Application number: 26164239.1
(22) Date of filing: 12.03.2026
(51) Int. Cl.: G05B 15/02

(54) **METHOD, DEVICE, SYSTEM, APPARATUS, PRODUCT, AND MEDIUM FOR HOME CARE**

(30) Priority: 24.03.2025 CN 202510353645
(71) Applicant: Robert Bosch GmbH, 70442 Stuttgart (DE)
(72) Inventor: ZHANG, Hansi, Shanghai, 200335 (CN); WANG, Asher, Shanghai, 200335 (CN)

(57) **Abstract**

The present disclosure relates to a method, an apparatus, a system, an electronic device, a computer program product, and a computer-readable storage medium for home care. The method comprises generating, by a first intelligent agent, a strategy for home care for a user in a current care cycle, based on first historical data and second historical data associated with the home care, the first historical data being generated prior to a current moment in the current care cycle, and the second historical data being generated in a plurality of historical care cycles. The method further comprises generating, by a second intelligent agent of a plurality of second intelligent agents, a task that may be performed by a tool of home care based on the strategy and the first historical data. Further, the method further comprises performing the task through the tool by a third intelligent agent of a plurality of third intelligent agents, which is associated with the second intelligent agent. According to embodiments of the present disclosure, complex strategic planning and generation of specific tasks can be performed outside of task execution, thereby adapting to complex and variable care scenarios.

## Description

### Technical Field

Embodiments of the present disclosure relate generally to the field of electronic devices, and in particular, to methods, apparatus, systems, electronic devices, computer program products, and computer-readable storage media for home care.

### Background

Smart home system is an intelligent, automated, networked, and integrated system that can provide users with a more convenient and comfortable living experience through technologies such as sensors, controllers, and algorithms. In smart home systems, a variety of smart devices and sensors can monitor home environmental parameters and user activity in real time, and analyze and process data through algorithms to control and optimize the home environment.

Smart home systems can be used for home care, such as home care associated with the elderly. Smart home systems can provide users in need of care with functions such as health monitoring and management, security monitoring, daily living assistance, and emotional communication and companionship. With the continuous development of smart home technology, smart home systems will occupy an even more important position in the field of home care.

### Summary of the Invention

Embodiments of the present disclosure provide a method, an apparatus, a system, an electronic device, a computer program product, and a computer-readable storage medium for home care.

In a first aspect of the present disclosure, a method for home care is provided. The method comprises generating, by a first intelligent agent, a strategy for home care of a user in a current care cycle, based on first historical data and second historical data associated with the home care, the first historical data being generated prior to a current moment in the current care cycle, the second historical data being generated in a plurality of historical care cycles. The method further comprises, by a second intelligent agent of a plurality of second intelligent agents, a task that may be performed by a tool of home care based on the strategy and the first historical data. Further, the method further comprises performing the task through the tool by a third intelligent agent of a plurality of third intelligent agents, which is associated with the second intelligent agent.

In a second aspect of the present disclosure, an apparatus for home care is provided. The apparatus comprises a strategy generation module configured to generate, by a first intelligent agent, a strategy for home care of a user in a current care cycle, based on first historical data and second historical data associated with the home care, the first historical data being generated prior to a current moment in the current care cycle, the second historical data being generated in a plurality of historical care cycles. The apparatus further comprises a task generation module configured to generate, by a second intelligent agent of a plurality of second intelligent agents, a task that may be performed by a tool of home care based on the strategy and the first historical data. Moreover, the apparatus further comprises a task execution module configured to perform the task through the tool by a third intelligent agent of a plurality of third intelligent agents, which is associated with the second intelligent agent.

In a third aspect of the present disclosure, a system for home care is provided, comprising a first intelligent agent, a plurality of second intelligent agents, and a plurality of third intelligent agents, wherein: the first intelligent agent is used to generate a strategy for home care of a user in a current care cycle, based on first historical data and second historical data associated with the home care, the first historical data being generated prior to a current moment in the current care cycle, the second historical data being generated in a plurality of historical care cycle; the second intelligent agent of the plurality of second intelligent agents is used to generate a task that may be performed by a tool of home care based on the strategy and the first historical data; and the third intelligent agent of the plurality of third intelligent agents, which is associated with the second intelligent agent, is used to perform the task through the tool.

In a fourth aspect of the present disclosure, an electronic device is provided. The electronic device comprises at least one processor. In addition, the electronic device further comprises a memory coupled to the at least one processor and having instructions stored thereon, wherein the instructions, when executed by the at least one processor, cause the electronic device to perform the method according to the first aspect of the present disclosure.

In a fifth aspect of the present disclosure, a computer program product is provided. The computer program product includes a computer program executed by a processor to implement the method provided according to the first aspect.

In a sixth aspect of the present disclosure, a computer-readable storage medium is provided. The computer-readable storage medium has computer-executable instructions stored thereon, wherein the computer-executable instructions are executed by a processor to implement the method provided according to the first aspect of the present disclosure.

It should be understood that the content described in the Summary of the Invention section is not intended to define the key or essential features of the embodiments of the present disclosure, nor is it intended to limit the scope of the present disclosure. Other features of the present disclosure will become readily understood by the following description.

### Brief Description of the Drawings

Above and other features, advantages and aspects of various embodiments of the present disclosure will become more apparent in combination with the accompanying drawings and with reference to the following detailed description. In the accompanying drawings, the same or similar reference numerals indicate the same or similar elements, wherein:
FIG. 1 shows a schematic diagram of an example environment in which some embodiments of the present disclosure may be implemented;
FIG. 2 shows a flow chart of a method for home care according to some embodiments of the present disclosure;
FIG. 3 shows a schematic diagram of an application scenario of a management intelligent agent according to some embodiments of the present disclosure;
FIG. 4 shows a schematic diagram of an application scenario of an expert intelligent agent according to some embodiments of the present disclosure;
FIG. 5 shows a schematic diagram of an application scenario of a working intelligent agent according to some embodiments of the present disclosure;
FIG. 6 shows a schematic diagram of a method for updating a task of home care according to some embodiments of the present disclosure;
FIG. 7A shows a schematic diagram of a process for updating a task of home care according to some embodiments of the present disclosure;
FIG. 7B shows a schematic diagram of another process for updating a task of home care according to some embodiments of the present disclosure;
FIG. 8 shows a block diagram of an apparatus for home care according to some embodiments of the present disclosure; and
FIG. 9 shows a schematic block diagram of an electronic device according to some embodiments of the present disclosure.

In all drawings, the same or similar reference numerals indicate the same or similar elements.

### Detailed Description of the Embodiments

The embodiments of the present disclosure will be described in further detail below with reference to the accompanying drawings. Although certain examples of the present disclosure are shown in the accompanying drawings, it should be understood that the present disclosure may be implemented in various forms and should not be construed as being limited to the examples set forth herein, rather these examples are provided for a more thorough and complete understanding of the present disclosure. It should be understood that the accompanying drawings and examples of the present disclosure are for exemplary purposes only and are not intended to limit the scope of protection of the present disclosure.

In the description of the examples disclosed herein, the term "comprise" and similar terms should be understood as open-ended inclusion, meaning "including but not limited to". The term "based on" should be understood as "at least partially based on." The term "one example" or "the example" should be understood as "at least one example." The terms "first," "second," etc., can refer to different or the same objects. Other explicit and implicit definitions may be included below.

As noted above, the smart home has important applications in the field of home care, especially in the field of home care for the elderly. The application of smart homes in the field of home care can provide users in need of home care with cares in areas such as health monitoring and management, security monitoring, daily living assistance, and emotional communication and companionship. In related technologies, the execution functions of various types of home care tasks are integrated into a software or hardware module in the smart home. However, related technologies are generally applicable to simple and fixed care scenarios. When complex and numerous care tasks are involved, it is difficult to plan and manage large-scale care tasks.

To this end, embodiments of the present disclosure provide a method for home care. The method involves three levels of intelligent agents, i.e., a first intelligent agent, a second intelligent agent, and a third intelligent agent. In the method, the first intelligent agent first plans a strategy for care of a user in a current care cycle based on short-term data and long-term data in a system; then the second intelligent agent generates a task to be performed by a tool of home care based on the planned strategy and the short-term data. Further, the third intelligent agent controls the tool of home care to perform the generated task.

In this way, the application of short-term and long-term data allows for more complex task planning by the first intelligent agent, and more targeted and time-sensitive task generation by the second intelligent agent, thereby dividing home care tasks into three levels of varying depths: task planning, task generation, and task execution. Thus, the embodiments of the present disclosure enable complex strategy planning and specific task generation beyond task execution, thereby adapting to complex and variable care scenarios.

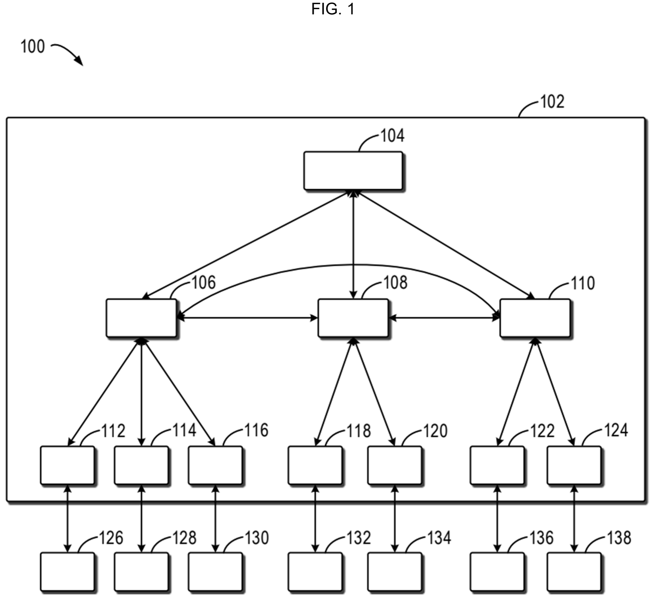

FIG. 1 shows a schematic diagram of an example environment 100 in which some embodiments of the present disclosure may be implemented. Referring to FIG. 1, the environment 100 includes a control center 102 for home care. In some embodiments, the control center 102 may include a user terminal, a mobile device, a computing machine, a computing system, a single server, a distributed server, or a cloud-based server. Alternatively, or additionally, the control center 102 may include an MCU (Micro Controller Unit), a CPU (Central Processing Unit), a GPU (Graphics Processing Unit), a GPGPU (General-purpose Computing on Graphics Processing Units), an FPGA (Field Programmable Gate Array), or other programmable logic devices, an ASIC (Application Specific Integrated Circuit), a discrete gate or a transistor logic device, discrete hardware components, etc.

It will be understood that the control center 102 of the present embodiment may be a single device as set forth above or a combination of any of the plurality of devices described above. For example, the control center 102 may be a central computer, a combination of a plurality of home care device controllers, or a combination of a central computer and a plurality of home care device controllers. There are no restrictions on the deployment method of the control center 102 in the present disclosure.

In some embodiments, the control center 102 is deployed with three levels of intelligent agents, including an intelligent agent 104 (which may be referred to as the first intelligent agent) in the first level, intelligent agents 106, 108, and 110 (which may be referred to as the second intelligent agents) in the second level, and intelligent agents 112, 114, 116, 118, 120, 122, and 124 in the third level. Here, the intelligent agents 112, 114, and 116 are associated with the intelligent agent 106, the intelligent agents 118, 120 are associated with the intelligent agent 108, and the intelligent agents 122, 124 are associated with the intelligent agent 110. It will be understood that the intelligent agent in the present embodiment is a large language model-based computer program with planning and thinking ability, memory capability, and the ability to use tool functions, and can complete a given task on its own.

In some embodiments, the control center 102 stores short-term data (which may be referred to as first historical data) and long-term data (which may be referred to as second historical data). The short-term data may be temporary data generated before the current moment in a current care cycle, including temporary operations, interaction information between intelligent agents, etc. For example, if a care cycle is one day, the short-term data is care-related operation and interaction information generated within that day prior to the current moment. The long-term data may be data generated in previous care cycles, such as care-related operation and interaction information for the previous three months. In some embodiments, the intelligent agent 104 may store short-term data and long-term data, while the intelligent agent 106, the intelligent agent 108, and the intelligent agent 110 store only short-term data. Alternatively, or in addition, the short-term data and the long-term data are stored independently, and the intelligent agent 104, the intelligent agent 106, the intelligent agent 108, and the intelligent agent 110 may access respective storage units to read the stored short-term data and long-term data.

In some embodiments, the intelligent agent 104 may generate a strategy for home care of a user in the current care cycle based on the short-term data and the long-term data, and send the strategy to the intelligent agent 106, the intelligent agent 108, and the intelligent agent 110. Then, the intelligent agent 106, the intelligent agent 108, and the intelligent agent 110 respectively generate home care tasks related to their functions based on the short-term data and the received strategy, and send the tasks to their respective intelligent agents. Further, upon receiving the tasks, the intelligent agent 112, the intelligent agent 114, the intelligent agent 116, the intelligent agent 118, the intelligent agent 120, the intelligent agent 122, and the intelligent agent 124 respectively control the corresponding home care tools to perform the received tasks. In some embodiments, a corresponding home care tool includes a tool 126, a tool 128, a tool 130, a tool 132, a tool 134, a tool 136, and a tool 138, respectively.

In the embodiments of the present disclosure, the application of short-term data and long-term data allows for more complex task planning by the intelligent agent 104, and more targeted and time-sensitive task generation by the intelligent agent 106, the intelligent agent 108, and the intelligent agent 110, thereby dividing home care tasks into three levels of varying depths: task planning, task generation, and task execution. Thus, embodiments of the present disclosure enable complex strategy planning and specific task generation beyond task execution, thereby adapting to complex and variable home care scenarios.

It should be understood that the architecture and functionality of the example environment 100 are described for exemplary purposes only and do not imply any limitation on the scope of the present disclosure. Embodiments of the present disclosure may also be applied to other environments having different structures and/or functions.

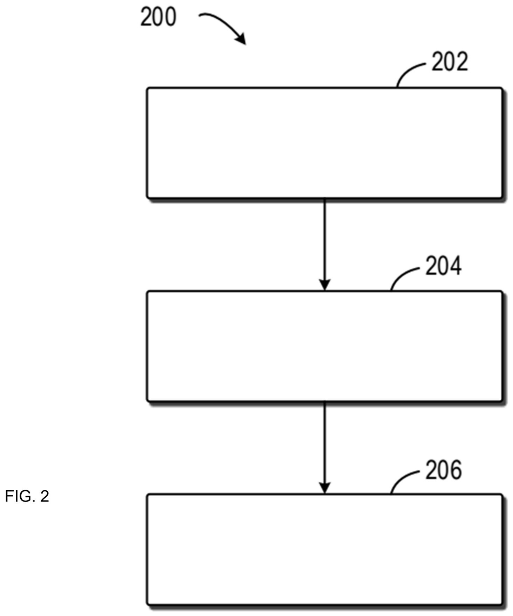

The process according to embodiments of the present disclosure will be described in detail below in conjunction with FIG. 2 to FIG. 9. For ease of understanding, the specific data mentioned in the following description are exemplary and are not used for defining the scope of protection of the present disclosure. It should be understood that the examples described below may also comprise additional actions not shown and/or actions shown may be omitted, and the scope of the present disclosure is not limited in this regard.

FIG. 2 shows a flow chart of a method 200 for home care according to some embodiments of the present disclosure. In some embodiments, in the example environment 100 shown in FIG. 1, the method 200 may be performed by the control center 102. It should be understood that although the following content is described with the control center 102 as the execution subject, the control center 200 may also be executed by other devices. The method 200 may further comprise additional actions not shown and/or actions shown may be omitted, the scope of the present disclosure being not limited in this regard.

At 202, a strategy for home care of a user in a current care cycle is generated by a first intelligent agent based on first historical data and second historical data associated with home care. The first historical data is generated prior to a current moment in the current care cycle, and the second historical data is generated in a plurality of historical care cycles. In some embodiments, the control center 102 may generate, by the intelligent agent 104 (which may be referred to as a first intelligent agent), a strategy for home care of a user in the current care cycle based on short-term data (which may be referred to as first historical data) and long-term data (which may be referred to as second historical data). Here, the short-term data may be temporary data generated before the current moment in the current care cycle, including temporary operations, interaction information between intelligent agents, etc. The long-term data may be data generated in a plurality of previous care cycles.

At 204, a second intelligent agent of a plurality of second intelligent agents may generate a task that may be performed by a tool of home care based on the strategy and the first historical data. In some embodiments, the intelligent agent 106, the intelligent agent 108, and the intelligent agent 110 (all of which may be referred to as the second intelligent agents) generate home care tasks related to their functions based on the short-term data and the received strategy, respectively, and the generated tasks may be performed by the home care tool.

At 206, the tasks are performed through a tool by a third intelligent agent of a plurality of third intelligent agents, which is associated with the second intelligent agent. In some embodiments, the intelligent agent 112, the intelligent agent 114, the intelligent agent 116 control a tool 126, a tool 128, and a tool 130 to perform the tasks generated by the intelligent agent 106, respectively. The intelligent agent 118 and the intelligent agent 120 control a tool 132, a tool 134, respectively, to perform tasks generated by the intelligent agent 108, and the intelligent agent 122 and the intelligent agent 124 control a tool 136 and a tool 138, respectively, to perform tasks generated by the intelligent agent 110.

**In** the embodiments of the present disclosure, the application of short-term and long-term data allows for more complex task planning by the first intelligent agent, and more targeted and time-sensitive task generation by the second intelligent agent, thereby dividing home care tasks into three levels of varying depths: task planning, task generation, and task execution. Thus, the embodiments of the present disclosure enable complex strategy planning and specific task generation beyond task execution, thereby adapting to complex and variable care scenarios.

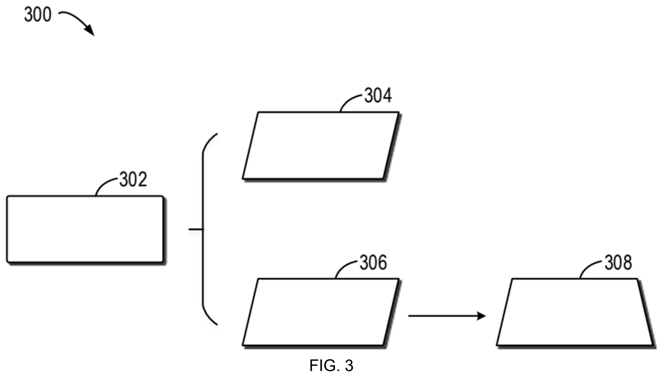

FIG. 3 shows a schematic view of an application scenario 300 of a working intelligent agent according to some embodiments of the present disclosure. In some embodiments, the working intelligent agent 302 (which may be referred to as a third intelligent agent) is a third-level intelligent agent that may be used to implement any one of the intelligent agent 112, the intelligent agent 114, the intelligent agent 116, the intelligent agent 118, the intelligent agent 120, the intelligent agent 122, and the intelligent agent 124. In some embodiments, the working intelligent agent 302 includes a role module 304 and a skill module 306. The role module 304 and the skill module 306 will be described in detail below.

In some embodiments, the role module 306 is used to define the role of the working intelligent agent 302 within the framework of its work, and the role of the working intelligent agent 302 in the present embodiment is to perform a specific task through a tool assigned thereto by the control center 102. In some embodiments, a skill in the skill module 306 may be used to implement a task assigned to the working intelligent agent 302, i.e., to control the tool 308 to perform the assigned task. In some embodiments, the tool 308 is used to perform tasks based on the control of the skill module 306.

In some embodiments, the working intelligent agent 302 may include a physical state intelligent agent, and the role module 304 thereof is used to define the role of the physical state intelligent agent as monitoring physical state data, such as blood pressure and heartbeat. The skill module 306 is used to implement skills such as monitoring, analyzing, and early warning of physical status data. The tool 308 includes a health monitoring device, data analysis software, an abnormal condition detection model, etc.

In some embodiments, the working intelligent agent 302 may include a pharmaceutical intelligent agent, and the role module 304 thereof is used to define the role of the pharmaceutical intelligent agent as managing medication plans, medical communication, etc. The skill module 306 includes skills for implementing medication management, etc. The tool 308 includes a dispenser, pharmacy application software and programs, etc.

In some embodiments, the working intelligent agent 302 may include a disease management intelligent agent, and the role module 304 thereof is used to define the role of the disease management intelligent agent as managing a variety of acute and chronic diseases, such as hypertension and diabetes. The skills module 306 includes skills for professional health management. The tool 308 includes a specific disease monitoring device, etc.

In some embodiments, the working intelligent agent 302 may include a nutrition intelligent agent, and the role module 304 thereof is used to define the role of the disease management intelligent agent as ensuring a nutritional balance in a user's diet. The skill module 306 includes skills for monitoring nutrient intake and nutritional analysis. The tool 308 includes a nutritional analysis model, a nutritional tracking application, and programs, etc.

In some embodiments, the working intelligent agent 302 may include a diet intelligent agent, and the role module 304 thereof is used to define the role of the diet intelligent agent as monitoring meal preparation and diet preparation. The skill module 306 includes skills for meal preparation and adjustment, monitoring intake per meal, etc. The tool 308 includes cooking utensils, a meal preparation application, etc.

In some embodiments, the working intelligent agent 302 may include an emotional intelligent agent, and the role module 304 thereof is to define the role of the emotional intelligent agent as monitoring and providing feedback on the user's emotional state, etc. The skill module 306 includes skills for a variety of emotional support. The tool 308 includes an emotion tracking software application and hardware devices (e.g., cameras, music players, desk lamps, etc.), an emotion detection model (or model interface), social media applications, and accounts.

In some embodiments, the working intelligent agent 302 may include an activity intelligent agent, and the role module 304 thereof is used to define the role of the activity intelligent agent as organizing social activities and entertainment, etc. The skill module 306 includes skills such as social activity organization and entertainment management. The tool 308 includes event planning applications, entertainment devices, social media applications, and accounts.

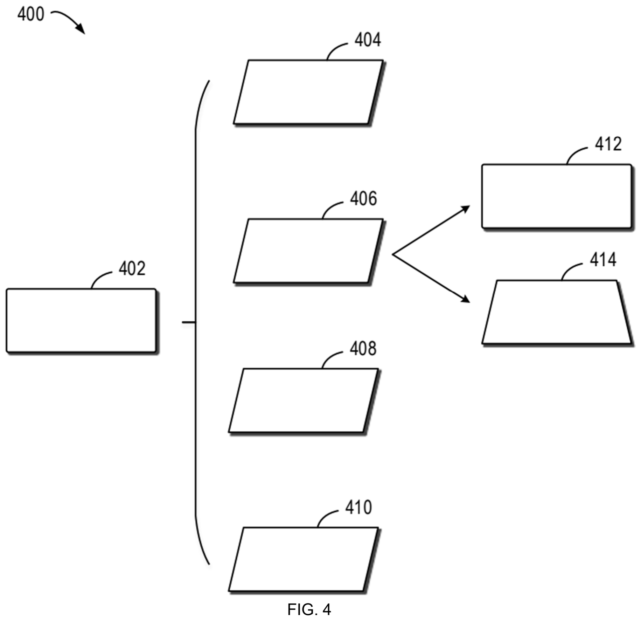

FIG. 4 shows a schematic view of an application scenario 400 of an expert intelligent agent according to some embodiments of the present disclosure. In some embodiments, the expert intelligent agent 402 (which may be referred to as a second intelligent agent) is a second-level intelligent agent that may be used to implement any one of the intelligent agent 106, the intelligent agent 108, and the intelligent agent 110. In some embodiments, the expert intelligent agent 402 includes a role module 404, a skill module 406, a planner 408, and short-term data 410. The role module 404, the skill module 406, the planner 408, and the short-term data 410 will be described in detail below. It will be understood that the expert intelligent agent 402 can communicate with the first-level intelligent agent, the second-level intelligent agent, and intelligent agents at the same level, i.e., the second-level intelligent agents.

In some embodiments, the role module 404 is used to define that the expert intelligent agent 402 can perform more complex tasks (as compared to the working intelligent agent 302), and it can inspect or organize the working intelligent agent by the short-term data 410. In some embodiments, the skills in the skill module 406 are more complex than the basic task execution (e.g., the skills in the skill module 304 in the working intelligent agent 302), enabling more complex activities. The skills in the skill module 406 may be implemented by the working intelligent agent 412 or by the tool 414.

In some embodiments, the planner 408 enables the expert intelligent agent 402 not only to perform tasks, but also to plan for various types of tasks according to a given strategy or a set of rules. The planned tasks may be performed by coordinating its skills. In some embodiments, unlike the working intelligent agent 302, the expert intelligent agent 402 also includes short-term data 410, that is, temporary data stored for a short-term in the current care cycle, allowing the expert intelligent agent 402 to store recent activities and communications, thereby enhancing the ability to make task decisions of the expert intelligent agent 402. The short-term data 410 may be deleted after the current care cycle ends.

In some embodiments, the expert intelligent agents may send interaction information (which may be referred to as the first interaction information) to each other. For example, an expert intelligent agent may inform other expert devices about the user's physical health status. In the control center 102, the expert intelligent agent 402 may receive interaction information (which may be referred to as the first interaction information) from other expert intelligent agents at the second level. The expert intelligent agent 402 may then generate and plan tasks according to the strategy sent by the first-level intelligent agent, i.e., the intelligent agent 104, short-term data 410, and the received interaction information. In this way, it is possible to combine interaction information between expert intelligent agents at the same level to generate tasks that need to be performed by the working intelligent agent, thus increasing the accuracy and user-specificity of the tasks.

With respect to the generation of the short-term data 410, in some embodiments, the control center 102 may acquire operations associated with tasks that have been performed during the current care cycle and obtain interaction information between the intelligent agents at three levels during the current care cycle. The control center 102 may then form the short-term data 410 for the current care cycle based on at least one of the aforementioned operations and the interaction information in the current care cycle. In some embodiments, the control center 102 may form the short-term data 410 based on interaction information between the intelligent agents at three levels when a care cycle has just begun (at this time no operation has been performed). In this way, the accuracy, comprehensiveness, and timeliness of the short-term data 410 can be improved.

In some embodiments, the above interaction information may be one or more of the interaction information between the second-level intelligent agents (which may be referred to as the first interaction information), i.e., expert intelligent agents, the interaction information between the first-level intelligent agents and the second-level intelligent agents (such as interaction information between the expert intelligent agents 402) (which may be referred to as the second interaction information), and the interaction information between the second-level intelligent agents and the third-level intelligent agents (which may be referred to as the third interaction information). The following will introduce the expert intelligent agent 402 based on a physical health intelligent agent, a nutrition and diet intelligent agent, and a mental health intelligent agent.

The physical health intelligent agent as the expert intelligent agent 402:
In some embodiments, the expert intelligent agent 402 may include a physical health intelligent agent. The role defined by the role module 404 may be used to manage the working intelligent agent to which it belongs (the physical state intelligent agent, the pharmaceutical intelligent agent, the disease management intelligent agent), summarize the physical health report, etc. The skill module 406 includes a skill of managing the working intelligent agents (the physical state intelligent agent, the pharmaceutical intelligent agent, the disease management intelligent agent), a skill of converting the task execution results of the working intelligent agents into physical health reports, and skills of accessing external knowledge bases to analyze physical health and generate expert-level recommendations, and communicating with other expert intelligent agents and the first-level intelligent agent 104.

In some embodiments, the control center 102 may perform the following steps with respect to the specific implementation of the above-mentioned skills: performing a task by the physical state intelligent agent, the pharmaceutical intelligent agent, and the disease management intelligent agent through a tool to generate a result associated with the physical health; generating, by the physical health intelligent agent, a physical health report for the user based on the result associated with the physical health; obtaining, by the physical health intelligent agent, physical health association information related to the physical health report from a knowledge base; generating, by the physical health intelligent agent, a recommendation related to physical health based on the physical health report, the physical health association information, and interaction information sent by other expert intelligent agents of a plurality of expert intelligent agents; and sending, by the physical health intelligent agent, a recommendation related to the physical health to the first-level intelligent agent 104 and the other intelligent agents. In this way, the intelligent agent 104 can update recommendations related to physical health into short-term data and long-term data for use in the generation of subsequent strategies, and other expert intelligent agents can also update recommendations related to physical health to short-term data for use in the generation of subsequent tasks. In this way, the accuracy of expert level recommendations related to physical health can be improved.

In some embodiments, the planner 408 may plan for daily health check tasks, medication plans, and disease management protocols, etc., and may make decisions on whether to update tasks, e.g., when a time point changes (at this time, it may communicate with the first-level intelligent agent 104) or when home care content changes (at this time, it may communicate with other expert intelligent agents). The short-term data 410 may include recent health events and medication management conditions, as well as interaction information obtained from communication with other intelligent agents (including other expert intelligent agents, the first-level intelligent agent 104, and the working intelligent agents to which they belong). The physical health intelligent agent may also perform some tasks through the tool 414, and the tool 414 may include external databases, report analysis tools, etc.

With respect to the nutrition and diet intelligent agent as the expert intelligent agent 402:
In some embodiments, the expert intelligent agent 402 may include a nutrition and diet intelligent agent. The role defined by the role module 404 can be used to manage the working intelligent agents to which it belongs (the nutrition intelligent agent, the diet intelligent agent), summarize the nutritional diet report, etc., to ensure that users, especially the elderly users, can achieve nutritional balance and proper diet plans. The skill module 406 includes a skill of managing the working intelligent agents (the nutrition intelligent agent, the diet intelligent agent), a skill of summarizing the task execution results of the working intelligent agents into nutritional diet reports, skills of accessing an external knowledge base to analyze the nutritional diet to generate expert-level recommendations, and communication with other expert intelligent agents and the first-level intelligent agent 104 (for example, interaction information of changes in health requirements, increased exercise, etc.).

In some embodiments, the control center 102 may perform the following steps with respect to the specific implementation of the above-mentioned skills: performing, by the nutrition intelligent agent and the diet intelligent agent, the task through the tool to generate a result associated with diet nutrition; generating, by the nutrition and diet intelligent agent, a nutritional diet report for the user based on the result associated with the diet nutrition; obtaining, by the nutrition and diet intelligent agent, nutritional diet association information related to the nutritional diet report from a knowledge base; generating, by the nutrition and diet intelligent agent, a recommendation related to a nutritional diet based on the nutritional diet report, the nutritional diet association information, and the interaction information sent by other expert intelligent agents of a plurality of expert intelligent agents; and sending, by the nutrition and diet intelligent agent, a recommendation related to a nutritional diet to the first-level intelligent agent 104 and other expert intelligent agents of the plurality of expert intelligent agents. In this way, the intelligent agent 104 can update recommendations related to nutritional diets into short-term data and long-term data for use in the generation of subsequent strategies, and other expert intelligent agents can also update recommendations related to nutritional diets to short-term data for use in the generation of subsequent tasks. In this way, the accuracy of expert level recommendations related to nutritional diets can be improved.

In some embodiments, the planner 408 may plan for daily nutrient intake and diet issues, and make decisions to update diet plans, such as communicating with the first-level intelligent agent 104 when the time point changes, and communicating with other expert intelligent agents when the nutritional diet content changes. The short-term data 410 may include recent dietary and nutritional changes, and interaction information such as health information recently obtained from other expert intelligent agents such as the physical health intelligent agent and the mental health intelligent agent. The nutrition and diet intelligent agent may also perform some tasks through the tool 414, and the tool 414 may include external databases, report analysis tools, etc.

With respect to the mental health intelligent agent as the expert intelligent agent 402:
In some embodiments, the expert intelligent agent 402 may include a mental health intelligent agent. The role defined by the role module 404 can be used to manage the working intelligent agents to which it belongs (the emotion intelligent agent, the activity intelligent agent), summarize the mental health report (including emotional health and social activity), etc. The skills module 406 includes a skill of managing the working intelligent agents (the emotion intelligent agent, the activity intelligent agent), skills of converting the task execution results of the working intelligent agents into mental health reports, accessing an external knowledge base to analyze mental health to generate expert-level recommendations, and communicating with other expert intelligent agents and the first-level intelligent agent 104.

In some embodiments, the control center 102 may perform the following steps with respect to the specific implementation of the above-mentioned skills: performing, by at least one of the emotion intelligent agent and the activity intelligent agent, the task through the tool to generate a result associated with mental health; generating, by the mental health intelligent agent, a mental health report for the user based on the result associated with the mental health; obtaining, by the mental health intelligent agent, mental health association information related to the mental health report from a knowledge base; generating, by the mental health intelligent agent, a recommendation related to mental health based on the mental health report, the mental health association information, and the interaction information sent by other expert intelligent agents of the plurality of expert intelligent agents; and sending, by the mental health intelligent agent, a recommendation related to mental health to the first-level intelligent agent104, and other expert intelligent agents of the plurality of expert intelligent agents. In this way, the intelligent agent 104 can update recommendations related to mental health into short-term data and long-term data for use in the generation of subsequent strategies, and other expert intelligent agents can also update recommendations related to mental health to short-term data for use in the generation of subsequent tasks. In this way, the accuracy of expert level recommendations related to mental health can be improved.

In some embodiments, the planner 408 may plan activities for a user to provide emotional support, organize social activities, etc. The short-term data 410 may include recent emotional states and social information, etc., as well as interaction information obtained from communication with other intelligent agents (including other expert intelligent agents, the first-level intelligent agent 104, and the working intelligent agents to which they belong). The mental health intelligent agent may also perform some tasks through the tool 414, and the tool 414 may include external databases, report analysis tools, etc.

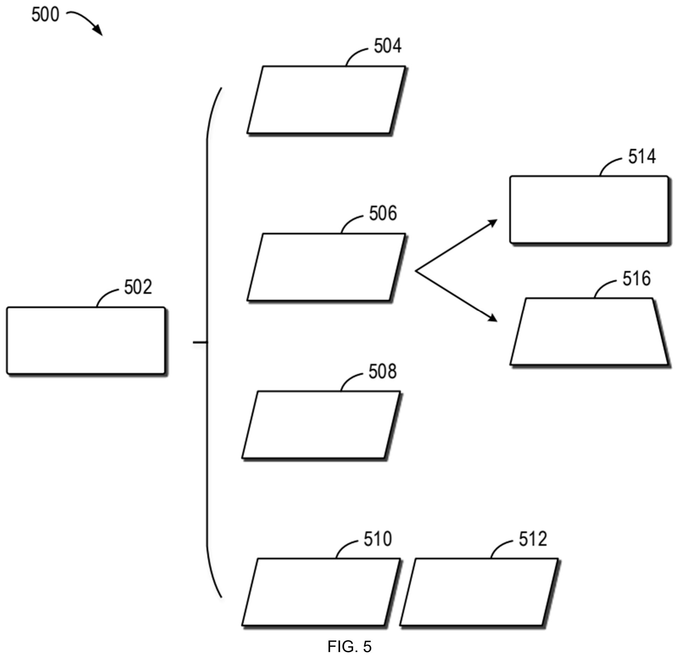

FIG. 5 shows a schematic diagram of an application scenario 500 of a management intelligent agent according to some embodiments of the present disclosure. In some embodiments, the management intelligent agent 502 (which may be referred to as a third intelligent agent) is a first-level intelligent agent that may be used to implement the intelligent agent 104. In some embodiments, the management intelligent agent 502 includes a role module 504, a skill module 506, a planner 508, short-term data 510, and long-term data 512. The role module 504, the skill module 506, the planner 508, the short-term data 510, and the long-term data 512 will be described in detail below.

In some embodiments, the role module 504 is used to define a central management role for home care that can oversee and manage all other agents in the control center 102, and ensure the implementation of complex operations and strategies. In some embodiments, the skill module 506 includes a skill of performing tasks (which may be performed by the tool 516), and skills of managing other intelligent agents (e.g., the working intelligent agent 514) for global strategy planning, etc.

In some embodiments, the planner 508 is capable of conducting complex strategy planning, leading to complex decision-making processes and the coordinated management of multiple intelligent agents and tasks. In some embodiments, the management intelligent agent 502 includes short-term data 510 and long-term data 512. The short-term data 510 and the long-term data 512 can provide guidance for strategy planning during the planning process. As such, the management intelligent agent 502 has access to a wide range of data, from instant details to historical content, enabling the management intelligent agent 502 to learn from current and past experiences during strategy planning.

In some embodiments, a daily life intelligent agent may act as the management intelligent agent 502. In the role module 504, the role of the daily life agent can be defined as the primary coordinator of multiple intelligent agents in the control center 102, thereby overseeing various aspects of the user's daily care. As a result, multiple specific working intelligent agents can be ensured to provide comprehensive support. The skill module 506 may include: management and coordination skills for managing multiple specific working intelligent agents to ensure seamless operation of various tasks; skills of making complex decisions for making strategy decisions based on the short-term data 510 and the long-term data 512; and self-adaptive adjustment capabilities for dynamically adjusting strategies based on user needs or the environment.

In some embodiments, the planner 508 in the daily life intelligent agent performs highly complex strategy planning and can dynamically plan to meet the needs of the user and home environment in detail. Specifically, the planner 508 may split a day into multiple time periods (e.g., 8 time periods) to plan strategies taking one time period as a unit. In this way, the strategy and ultimately generated tasks can be guaranteed to be more accurate and time-sensitive. The planner 508 may provide for step-by-step coordination, and uniformly manage specific working agents to perform tasks, such as through the Stanford Small Town Approach. In this way, various types of tasks can be carried out step by step to cover all aspects of daily life. The planner 508 can adjust strategies dynamically by continuously monitoring the user's status and adjusting strategies in real time, thus adapting to various changes and unexpected events.

In some embodiments, the short-term data 508 may include recent user activities that are continuously tracked, and the short-term data 508 can be dynamically adjusted by the control center 102 to ensure that the user's immediate needs are satisfied. In some embodiments, the long-term data 510 may include historically generated data, including but not limited to user profiles (diseases, medications, dietary habits, etc.) and periodic inspection results. The long-term data 510 can enhance the ability to plan for future strategies. In this way, the daily life intelligent agent can learn from historical experiences to plan care strategies.

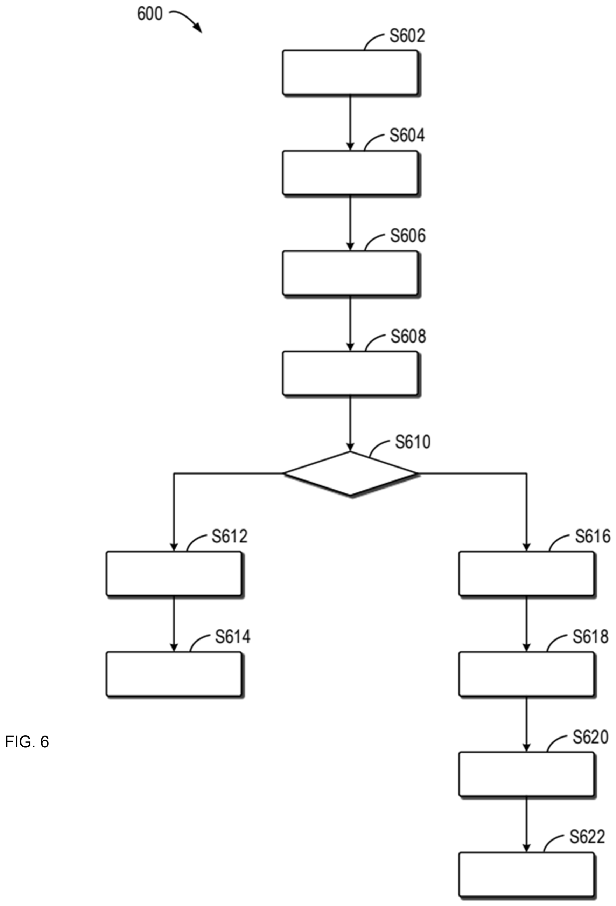

FIG. 6 shows a schematic diagram of a method 600 for updating a task of home care according to some embodiments of the present disclosure. In some examples, in the example environment 100 shown in FIG. 1, the method 600 may be performed by the control center 102. It should be understood that although the following content is described with the control center 102 as the execution subject, the method 600 may also be executed by other devices. The method 600 may further comprise additional actions not shown and/or actions shown may be omitted, and the scope of the present disclosure is not limited in this regard.

At S602, the working intelligent agent acquires status data of the user. In some embodiments, the working intelligent agent (which may be referred to as the third intelligent agent) in the control center 102 executes a monitoring task through a monitoring tool to obtain status data of the user, such as data of a user's heart rate, blood pressure, blood oxygen saturation, etc. At S604, the working intelligent agent alerts the expert intelligent agent (which may be referred to as the second intelligent agent). In some embodiments, the working intelligent agent sends the status data to the expert intelligent agent upon detecting the status data.

At S606, the expert intelligent agent generates a recommendation for task changes. In some embodiments, upon receiving the status data of the user, the expert intelligent agent may generate a recommendation related to task changes based on the status data. At S608, the management intelligent agent (which may be referred to as the first intelligent agent) generates a decision on whether to adjust the strategy. In some embodiments, the management intelligent agent may generate a decision on whether to adjust a current care strategy based on the status data of the user and recommendations generated by the expert intelligent agent. At S610, the management intelligent agent determines whether the decision is to adjust the strategy.

If the decision is to adjust the strategy, at S612, the management intelligent agent adjusts the strategy based on the status data and distributes the strategy to the expert intelligent agent. In some embodiments, if the management intelligent agent decides to adjust the strategy, the strategy may be adjusted based on the latest status data and distributed to the next-level expert intelligent agent for more detailed task planning. At S614, the expert intelligent agent adjusts the task according to the adjusted strategy and uploads the task to the management intelligent agent. In some embodiments, upon receipt of the adjusted strategy, the expert intelligent agent adjusts the content of the task according to the adjusted strategy and uploads it to the management intelligent agent. In this way, the management intelligent agent can update short-term data and long-term data based on the adjusted task for use in the generation of subsequent strategy. In addition, the expert intelligent agent can also distribute the adjusted tasks to the working intelligent agent, so that the working intelligent agent controls the relevant tools to perform the tasks. This allows for timely adjustment of the strategy and updating of the executed care tasks when the user's state changes, thereby improving the user experience.

If the decision is not to adjust the strategy, at S616, the management intelligent agent sends an instruction to the expert intelligent agent not to adjust the strategy. At S618, the expert intelligent agent informs other expert intelligent agents of the instruction. In some embodiments, if the management intelligent agent decides not to adjust the strategy, relevant instructions of the management intelligent agent may be sent to other expert intelligent agents to inform the other expert intelligent agents that the current care strategy does not need to be adjusted. At S620, the expert intelligent agent searches the knowledge base for task planning. In some embodiments, the expert intelligent agent may obtain relevant information and planning for tasks from a knowledge base related to heart rate, a knowledge base related to blood oxygen, a knowledge base related to blood pressure, etc. At S622, the expert intelligent agent adjusts the task and uploads it to the management intelligent agent. In some embodiments, the expert intelligent agent adjusts the task content and uploads it to the management intelligent agent based on the information obtained from the knowledge base. In this way, the management intelligent agent can update short-term data and long-term data based on the adjusted tasks for use in the generation of subsequent strategy. In addition, the expert intelligent agent can also distribute the adjusted tasks to the working intelligent agent, so that the work intelligent agent controls the relevant tools to perform the tasks. In this way, it is possible to locally update the care tasks performed without adjusting the overall strategy, thereby improving the user experience.

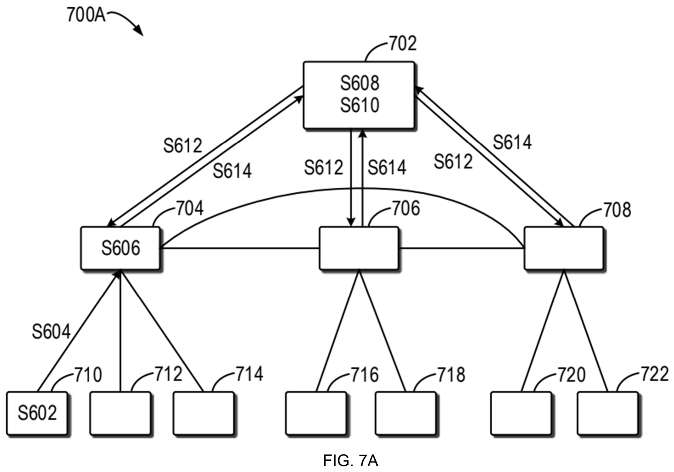

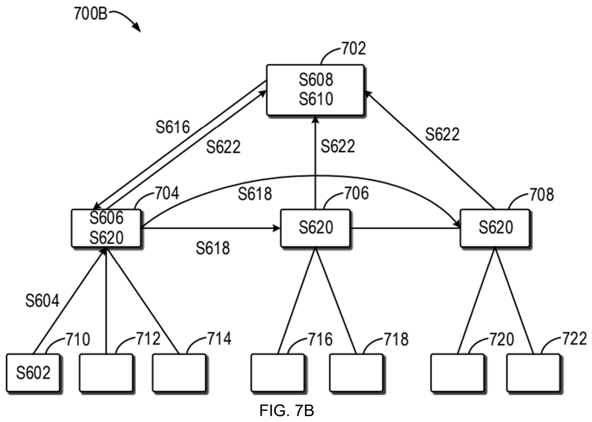

FIG. 7A shows a schematic diagram of a process 700A for updating a task of home care according to some embodiments of the present disclosure, and FIG. 7B shows a schematic diagram of another process 700B for updating a task of home care according to some embodiments of the present disclosure. In some embodiments, the management intelligent agent includes a daily life intelligent agent 702, and the expert intelligent agent includes a physical health intelligent agent 704, a nutrition and diet intelligent agent 706, and a mental health intelligent agent 708, and the working intelligent agent includes a physical state intelligent agent 710, a pharmaceutical intelligent agent 712, a disease management intelligent agent 714, a nutrition intelligent agent 716, a diet intelligent agent 718, an emotion intelligent agent 720, and an activity intelligent agent 722.

In some embodiments, referring to FIG. 7A, at S602, the physical state intelligent agent 710 obtains status data of a user. At S604, the physical state intelligent agent 710 alerts the physical health intelligent agent 704. At S606, the physical health intelligent agent 704 generates a recommendation for task changes. At S608, the daily life intelligent agent 702 generates a decision on whether to adjust the strategy. At S610, the daily life intelligent agent 702 determines whether the decision is to adjust the strategy. If it is decided to adjust the strategy, then at S612, the daily life intelligent agent 702 adjusts the strategy based on the status data and distributes it to the physical health intelligent agent 704, the nutrition and diet intelligent agent 706, and the mental health intelligent agent 708. At S614, the physical health intelligent agent 704, the nutrition and diet intelligent agent 706, and the mental health intelligent agent 708 adjust the tasks and upload the tasks to the daily life intelligent agent 702.

In some embodiments, referring to FIG. 7B, at S602, the physical state intelligent agent 710 obtains status data of a user. At S604, the physical state intelligent agent 710 alerts the physical health intelligent agent 704. At S606, the physical health intelligent agent 704 generates a recommendation for task changes. At S608, the daily life intelligent agent 702 generates a decision on whether to adjust the strategy. At S610, the daily life intelligent agent 702 determines whether the decision is to adjust the strategy. If the decision is not to adjust the strategy, then at S616, the daily life intelligent agent 702 sends an instruction to the physical health intelligent agent 704 not to adjust the strategy. At S618, the physical health intelligent agent 704 informs the nutrition and diet intelligent agent 706 and the mental health intelligent agent 708 of the instruction. At S620, the physical health intelligent agent 704, the nutrition and diet intelligent agent 706, and the mental health intelligent agent 708 search a knowledge base for task planning. In S622, the physical health intelligent agent 704, the nutrition and diet intelligent agent 706, and the mental health intelligent agent 708 adjust the tasks and upload the tasks to the daily life intelligent agent 702.

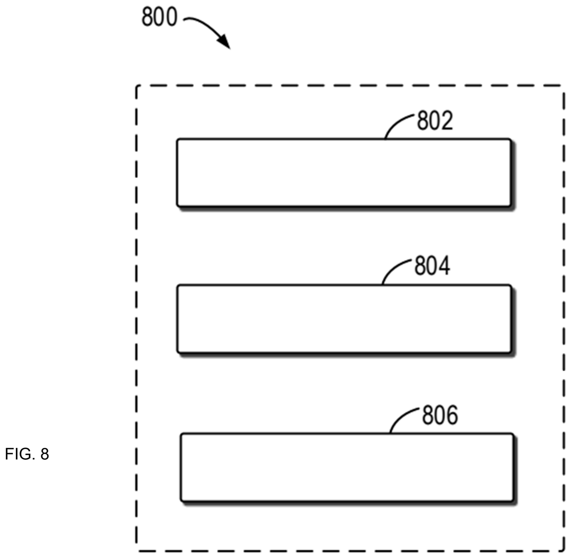

FIG. 8 shows a block diagram of an apparatus 800 for home care according to some embodiments of the present disclosure. The apparatus 800 includes a strategy generation module 802, configured to generate, by a first intelligent agent, a strategy for home care of a user in a current care cycle, based on first historical data and second historical data associated with the home care, the first historical data being generated prior to a current moment in the current care cycle, the second historical data being generated in a plurality of historical care cycles. The apparatus 800 also includes a task generation module 804, configured to generate, by a second intelligent agent of a plurality of second intelligent agents, a task that may be performed by a tool of home care based on the strategy and the first historical data. Further, the apparatus 800 further includes a task execution module 806, configured to perform the task through the tool by a third intelligent agent of a plurality of third intelligent agents, which is associated with the second intelligent agent.

In some embodiments, the task generation module 804 is further configured to: obtain, by a second intelligent agent, first interaction information from other second intelligent agents of the plurality of second intelligent agents; and generate, by the second intelligent agent, a task based on the first interaction information, the strategy, and the first historical data.

In some examples, the apparatus 800 is further configured to: obtain an operation associated with a historical task within the current care cycle; obtain interaction information of the first intelligent agent, the plurality of second intelligent agents, and the plurality of third intelligent agents within the current care cycle, the interaction information including at least one of a first interaction information between the plurality of second intelligent agents, a second interaction information between the first intelligent agent and the plurality of second intelligent agents, and a third interaction information between the plurality of second intelligent agents and the plurality of third intelligent agents; and store the operation and/ or the interaction information within the current care cycle as the first historical data within the current care cycle.

In some embodiments, the task is used to acquire status data of the user, and the apparatus 800 is further configured to: obtain, by the third intelligent agent, the status data of the user through the tool; generate, by the second intelligent agent, a recommendation whether to adjust the strategy based on the status data; generate, by the first intelligent agent, a decision on whether to adjust the strategy based on the recommendation and the status data; and adjust, by the first intelligent agent, the strategy based on the status data in response to the decision instructing adjustment of the strategy.

In some examples, the apparatus 800 is further configured to: send, by the first intelligent agent, an adjusted strategy to the plurality of second intelligent agents; adjust, by the second intelligent agents, the task performed by the tool based on the adjusted strategy; and send, by the second intelligent agent, an adjusted task to the first intelligent agent and/or the plurality of third intelligent agents to store the adjusted task by the first intelligent agent and/or perform the adjusted task by the plurality of third intelligent agents through the tool.

In some examples, the apparatus 800 is further configured to: send, by the first intelligent agent to the second intelligent agent, an instruction associated with the decision in response to the decision instructing not to adjust the strategy; send, by the second intelligent agent, the instruction to other second intelligent agents of the plurality of second intelligent agents; obtain, by the plurality of second intelligent agents, association information of the status data from a database associated with the status data; adjust, by the plurality of second intelligent agents, the task performed by the tool based on the status data and the association information; and send, by the plurality of second intelligent agents to the first intelligent agent and/or the third intelligent agents, a plurality of adjusted tasks to store the plurality of adjusted tasks by the first intelligent agent and/or perform the plurality of adjusted tasks by the plurality of third intelligent agents through the tool.

In some embodiments, the second intelligent agent includes a physical health intelligent agent, the third intelligent agent associated with the physical health intelligent agent includes at least one of a physical state intelligent agent, a pharmaceutical intelligent agent, and a disease management intelligent agent, and the apparatus 800 is further configured to: perform the task by at least one of the physical state intelligent agent, the pharmaceutical intelligent agent, and the disease management intelligent agent through the tool to generate a result associated with the physical health; generate, by the physical health intelligent agent, a physical health report for the user based on the result associated with the physical health; obtain, by the physical health intelligent agent, physical health association information related to the physical health report from a knowledge base; generate, by the physical health intelligent agent, a recommendation related to physical health based on the physical health report, the physical health association information, and a first interchange information sent by other second intelligent agents of the plurality of second intelligent agents; and send, by the physical health intelligent agent, a recommendation related to the physical health to at least one of the first intelligent agent and the other second intelligent agents of the plurality of second intelligent agents.

In some embodiments, the second intelligent agent includes a nutrition and diet intelligent agent, the third intelligent agent associated with the nutrition and diet intelligent agent includes at least one of the nutrition intelligent agent and the diet intelligent agent, and the apparatus 800 is further configured to: perform, by at least one of the nutrition intelligent agent and the diet intelligent agent, the task through the tool to generate a result associated with diet nutrition; generate, by the nutrition and diet intelligent agent, a nutritional diet report for the user based on the result associated with the diet nutrition; obtain, by the nutrition and diet intelligent agent, nutritional diet association information related to the nutritional diet report from a knowledge base; generate, by the nutrition and diet intelligent agent, a recommendation related to a nutritional diet based on the nutritional diet report, the nutritional diet association information, and the first interaction information sent by other second intelligent agents of the plurality of second intelligent agents; and send, by the nutrition and diet intelligent agent, a recommendation related to a nutritional diet to at least one of the first intelligent agent and the other second intelligent agents of the plurality of second intelligent agents.

In some embodiments, the second intelligent agent includes a mental health intelligent agent, the third intelligent agent associated with the mental health intelligent agent includes at least one of an emotion intelligent agent and an activity intelligent agent, and the apparatus 800 is further configured to: perform, by at least one of the emotion intelligent agent and the activity intelligent agent, the task through the tool to generate a result associated with mental health; generate, by the mental health intelligent agent, a mental health report for the user based on the result associated with the mental health; obtain, by the mental health intelligent agent, mental health association information related to the mental health report from a knowledge base; generate, by the mental health intelligent agent, a recommendation related to mental health based on the mental health report, the mental health association information, and the first interaction information sent by other second intelligent agents of the plurality of second intelligent agents; and send, by the mental health intelligent agent, a recommendation related to mental health to at least one of the first intelligent agent and the other second intelligent agents of the plurality of second intelligent agents.

It will be understood that the apparatus 800 of the present disclosure may achieve at least one of a number of advantages that the method or process described above can achieve. For example, the apparatus 800 allows for, based on the application of short-term data and long-term data, more complex task planning by the first intelligent agent, and more targeted and time-sensitive task generation by the second intelligent agent, thereby dividing home care tasks into three levels of varying depths: task planning, task generation, and task execution. Thus, embodiments of the present disclosure enable complex strategy planning and specific task generation beyond task execution, thereby adapting to complex and variable care scenarios.

In some embodiments, a system for home care is provided, comprising a first intelligent agent, a plurality of second intelligent agents, and a plurality of third intelligent agents, wherein: the first intelligent agent is used to generate a strategy for home care of a user in a current care cycle, based on first historical data and second historical data associated with the home care, the first historical data being generated prior to a current moment in the current care cycle, the second historical data being generated in a plurality of historical care cycle; the second intelligent agent of the plurality of second intelligent agents is used to generate a task that may be performed by a tool of home care based on the strategy and the first historical data; and the third intelligent agent of the plurality of third intelligent agents, which is associated with the second intelligent agent, is used to perform the task through the tool. The first intelligent agent, the second intelligent agent, and the third intelligent agent have been described in detail in the above embodiments, and will not be described in detail here.

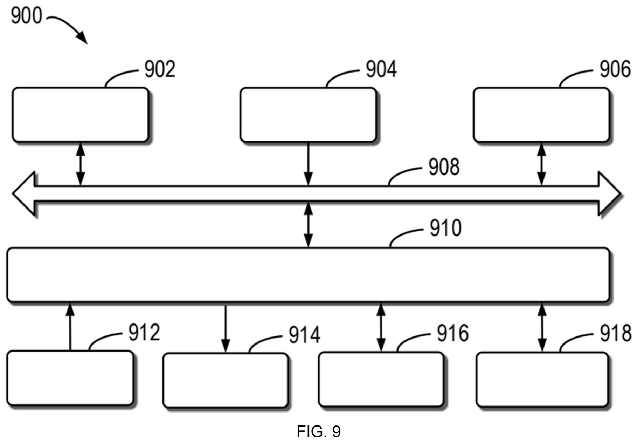

FIG. 9 shows a block diagram of an electronic device 900 according to certain embodiments of the present disclosure. The electronic device 900 may be a device or an apparatus described in the embodiments of the present disclosure. As shown in FIG. 9, the electronic device 900 includes a central processing unit (CPU) and /or a graphics processing unit (GPU) 902 that may perform various appropriate actions and processing according to computer program instructions stored in read-only memory (ROM) 904 or computer program instructions loaded into random access memory (RAM) 906 from a storage unit 916. Various programs and data required for the operation of the electronic device 900 may also be stored in the RAM 906. The CPU/GPU 902, the ROM 904, and the RAM 906 are interconnected through a bus 908. An input/output (I/O) interface 910 is also connected to the bus 908. Although not shown in FIG. 9, the electronic device 900 may also include a coordination processor.

A plurality of components in the electronic device 900 are connected to the I/O interface 910, comprising: an input unit 912, such as a keyboard and a mouse; an output unit 914, such as various types of display and speakers; a storage unit 916, such as a disk and optical discs; as well as a communication unit 918, such as a network interface card, a modem, and a wireless communication transceiver. The communication unit 918 allows the electronic device 900 to exchange informatior/data with other devices through computer networks such as the Internet and /or various telecommunication networks.

The various methods or processes described above may be performed by the CPU/GPU 902. For example, in some embodiments, the method can be implemented as a computer software program tangibly contained in a machine-readable medium, such as the storage unit 916. In some examples, part or all of the computer programs may be loaded and/or installed onto the electronic device 900 through the ROM 904 and/or the communication unit 918. When the computer program is loaded onto the RAM 906 and executed by the CPU/GPU 902, one or more steps or actions in the methods or processes described above may be performed.

In some embodiments, the methods and processes described above may be implemented as computer program products. The computer program product may comprise a computer-readable storage medium uploaded with computer-readable program instructions for performing various aspects of the present disclosure.

The computer-readable storage medium may be a tangible device that maintains and stores instructions used by instruction execution devices. The computer-readable storage medium may be, for example, but not limited to, an electrical storage device, a magnetic storage device, an optical storage device, an electromagnetic storage device, a semiconductor memory device, or any suitable combination of the above. More specific examples of the computer-readable storage medium (a non-exhaustive list) comprise: a portable computer disc, a hard disc, a random access memory (RAM), a read-only memory (ROM), an erasable programmable read-only memory (EPROM or flash), a static random access memory (SRAM), a portable compact disc read-only memory (CD-ROM), a digital versatile disc (DVD), a memory stick, a floppy disc, a mechanical coder, such as a punch card with instructions or structures with protrusions in grooves or indentations, as well as any suitable combinations of the above. The computer-readable storage medium used herein is not to be construed as transient signals per se, such as radio waves or other freely propagating electromagnetic waves, electromagnetic waves propagating through waveguides or other transmission media (e.g., light pulses through fiber optic cables), or electrical signals transmitted through wires.

The computer-readable program instructions described herein can be downloaded to respective computing/processing devices from a computer-readable storage medium or to an external computer or external storage device via a network, for example, the Internet, a local area network, a wide area network, and/or a wireless network. The network can comprise copper transmission cables, fiber optic transmission, wireless transmission, routers, firewalls, switches, gateway computers, and/or edge servers. A network adapter card or network interface in each computing/processing device receives computer-readable program instructions from the network and forwards the computer-readable program instructions for storage in a computer-readable storage medium within the respective computing/processing device.

The computer program instructions used to perform the operations of the present disclosure may be assembly instructions, instruction set architecture (ISA) instructions, machine instructions, machine-related instructions, microcode, firmware instructions, state set data, or source code or object code written with any combination of one or more programming languages. The programming language includes object-oriented programming languages as well as conventional procedural programming languages. Computer-readable program instructions may be fully executed on the user's computer, partially executed on the user's computer, executed as an independent software package, partially executed on the user's computer and partially executed on a remote computer, or fully executed on a remote computer or server. In cases involving remote computers, the remote computer can be connected to the user's computer through any type of network-including a local area network (LAN) or a wide area network (WAN)-or can be connected to an external computer (for example, through the Internet using an Internet Service Provider). In some examples, electronic circuits, such as programmable logic circuits, field-programmable gate arrays (FPGA), or programmable logic arrays (PLA), can be personalized by utilizing state information of the computer-readable program instructions, which can execute the computer-readable program instructions to implement various aspects of the present disclosure.

These computer-readable program instructions may be provided to general-purpose computers, dedicated computers or the processing units of other programmable data processing devices, thereby producing a type of machine, such that when these instructions are executed by the computers or processing units of other programmable data processing devices, an apparatus that realizes the functions/actions stipulated in one or more blocks in the flow charts and/or block diagrams is produced. These computer-readable program instructions may also be stored in computer-readable storage medium, enabling computers, programmable data processing devices, and/or other devices to operate in a specific manner. Therefore, the computer-readable media containing instructions comprise a manufactured product that includes instructions for implementing various aspects of the functions/actions specified in one or more blocks in the flow charts and/or block diagrams.

The computer-readable program instructions may also be loaded onto a computer, other programmable data processing devices, or other devices, enabling a series of operational steps to be executed on the computer, other programmable data processing devices, or other devices to generate a computer-implemented process. This enables the instructions executed on the computer, other programmable data processing devices, or other devices to implement the functions/actions specified in one or more blocks in the flow charts and/or block diagrams.

The flow charts and block diagrams in the accompanying drawings illustrate possible system architectures, functionalities, and operations for devices, methods, and computer program products according to multiple embodiments of the present disclosure. Regarding this, every block in the flow chart or block diagram can represent a part of a module, program section or instructions, wherein the part of the module, program section or instructions contains one or a plurality of executable instructions that are used to implement the stipulated logic function. In some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It must also be noted that every block in the block diagrams and/or flow charts, as well as combinations of blocks in the block diagrams and/or flow charts may be implemented by dedicated hardware-based systems used to perform the stipulated functions or actions, or implemented by using combinations of dedicated hardware and computer instructions.

The above description of the various embodiments of the present disclosure is exemplary and not intended to be exhaustive or limited to the disclosed embodiments. Many modifications and variations will be apparent to those of ordinary skill in the art without departing from the scope and spirit of the described embodiments. The terms used herein are chosen to best explain the principles and practical application of the various examples or the technological improvements in the technology in the market, or allow others of ordinary skill in the art to understand the various examples disclosed herein.

## Claims

1. A method (200) for home care, comprising:
generating (202), by a first intelligent agent, a strategy for home care of a user in a current care cycle, based on first historical data and second historical data associated with the home care, the first historical data being generated prior to a current moment in the current care cycle, the second historical data being generated in a plurality of historical care cycles;
generating (204), by a second intelligent agent of a plurality of second intelligent agents, a task that may be performed by a tool of home care based on the strategy and the first historical data; and
performing (206) the task through the tool by a third intelligent agent of a plurality of third intelligent agents, which is associated with the second intelligent agent.

2. The method (200) according to claim 1, wherein generating (204) the task comprises:
obtaining, by the second intelligent agent, first interaction information from other second intelligent agents of the plurality of second intelligent agents; and
generating, by the second intelligent agent, the task based on the first interaction information, the strategy, and the first historical data.

3. The method (200) according to claim 1 or 2, further comprising:
obtaining an operation associated with a historical task within the current care cycle;
obtaining interaction information of the first intelligent agent, the plurality of second intelligent agents, and the plurality of third intelligent agents within the current care cycle, the interaction information including at least one of a first interaction information between the plurality of second intelligent agents, a second interaction information between the first intelligent agent and the plurality of second intelligent agents, and a third interaction information between the plurality of second intelligent agents and the plurality of third intelligent agents; and
storing the operation and/or the interaction information within the current care cycle as the first historical data within the current care cycle.

4. The method (200) according to claim 1, wherein the task is used to acquire status data of the user, and the method further comprises:
obtaining, by the third intelligent agent, the status data of the user through the tool;
generating, by the second intelligent agent, a recommendation whether to adjust the strategy based on the status data;
generating, by the first intelligent agent, a decision on whether to adjust the strategy based on the recommendation and the status data; and
adjusting, by the first intelligent agent, the strategy based on the status data in response to the decision instructing adjustment of the strategy.

5. The method (200) according to claim 4, further comprising:
sending, by the first intelligent agent, an adjusted strategy to the plurality of second intelligent agents;
adjusting, by the second intelligent agents, the task performed by the tool based on the adjusted strategy; and
sending, by the second intelligent agent, an adjusted task to the first intelligent agent and/or the plurality of third intelligent agents to store the adjusted task by the first intelligent agent and/or perform the adjusted task by the plurality of third intelligent agents through the tool.

6. The method (200) according to claim 4, further comprising:
sending, by the first intelligent agent to the second intelligent agent, an instruction associated with the decision in response to the decision instructing not to adjust the strategy;
sending, by the second intelligent agent, the instruction to other second intelligent agents of the plurality of second intelligent agents;
obtaining, by the plurality of second intelligent agents, association information of the status data from a database associated with the status data;
adjusting, by the plurality of second intelligent agents, the task performed by the tool based on the status data and the association information; and
sending, by the plurality of second intelligent agents to the first intelligent agent and/ or the third intelligent agents, a plurality of adjusted tasks to store the plurality of adjusted tasks by the first intelligent agent and/or perform the plurality of adjusted tasks by the plurality of third intelligent agents through the tool.

7. The method (200) according to any one of claims 1 to 6, wherein the second intelligent agent includes a physical health intelligent agent, the third intelligent agent associated with the physical health intelligent agent includes at least one of a physical state intelligent agent, a pharmaceutical intelligent agent, and a disease management intelligent agent, and the method further comprises:
performing the task by at least one of the physical state intelligent agent, the pharmaceutical intelligent agent, and the disease management intelligent agent through the tool to generate a result associated with the physical health;
generating, by the physical health intelligent agent, a physical health report for the user based on the result associated with the physical health;
obtaining, by the physical health intelligent agent, physical health association information related to the physical health report from a knowledge base;
generating, by the physical health intelligent agent, a recommendation related to physical health based on the physical health report, the physical health association information, and a first interaction information sent by other second intelligent agents of the plurality of second intelligent agents; and
sending, by the physical health intelligent agent, a recommendation related to the physical health to at least one of the first intelligent agent and the other second intelligent agents of the plurality of second intelligent agents.

8. The method (200) according to any one of claims 1 to 6, wherein the second intelligent agent comprises a nutrition and diet intelligent agent, a third intelligent agent associated with the nutrition and diet intelligent agent comprises at least one of a nutrition intelligent agent and a diet intelligent agent, and the method further comprises:
performing, by at least one of the nutrition intelligent agent and the diet intelligent agent, the task through the tool to generate a result associated with diet nutrition;
generating, by the nutrition and diet intelligent agent, a nutritional diet report for the user based on the result associated with the diet nutrition;
obtaining, by the nutrition and diet intelligent agent, nutritional diet association information related to the nutritional diet report from a knowledge base;
generating, by the nutrition and diet intelligent agent, a recommendation related to a nutritional diet based on the nutritional diet report, the nutritional diet association information, and the first interaction information sent by other second intelligent agents of the plurality of second intelligent agents; and
sending, by the nutrition and diet intelligent agent, a recommendation related to a nutritional diet to at least one of the first intelligent agent and the other second intelligent agents of the plurality of second intelligent agents.

9. The method (200) according to any one of claims 1 to 6, wherein the second intelligent agent comprises a mental health intelligent agent, a third intelligent agent associated with the mental health intelligent agent comprises at least one of an emotion intelligent agent and an activity intelligent agent, and the method further comprises:
performing, by at least one of the emotion intelligent agent and the activity intelligent agent, the task through the tool to generate a result associated with mental health;
generating, by the mental health intelligent agent, a mental health report for the user based on the result associated with the mental health;
obtaining, by the mental health intelligent agent, mental health association information related to the mental health report from a knowledge base;
generating, by the mental health intelligent agent, a recommendation related to mental health based on the mental health report, the mental health association information, and the first interaction information sent by other second intelligent agents of the plurality of second intelligent agents; and
sending, by the mental health intelligent agent, a recommendation related to mental health to at least one of the first intelligent agent and the other second intelligent agents of the plurality of second intelligent agents.

10. An apparatus (800) for home care, comprising:
a strategy generation module (802), configured to generate, by a first intelligent agent, a strategy for home care of a user in a current care cycle, based on first historical data and second historical data associated with the home care, the first historical data being generated prior to a current moment in the current care cycle, the second historical data being generated in a plurality of historical care cycles;
a task generation module (804), configured to generate, by a second intelligent agent of a plurality of second intelligent agents, a task that may be performed by a tool of home care based on the strategy and the first historical data; and
a task execution module (806), configured to perform the task through the tool by a third intelligent agent of a plurality of third intelligent agents, which is associated with the second intelligent agent.

11. A system for home care, comprising a first intelligent agent, a plurality of second intelligent agents, and a plurality of third intelligent agents, wherein:
the first intelligent agent is used to generate a strategy for home care of a user in a current care cycle, based on first historical data and second historical data associated with the home care, the first historical data being generated prior to a current moment in the current care cycle, the second historical data being generated in a plurality of historical care cycle;
the second intelligent agent of the plurality of second intelligent agents is used to generate a task that may be performed by a tool of home care based on the strategy and the first historical data; and
the third intelligent agent of the plurality of third intelligent agents, which is associated with the second intelligent agent, is used to perform the task through the tool.

12. An electronic device (900), comprising:
at least one processor, and
a memory coupled to the at least one processor and having instructions stored thereon, wherein the instructions, when executed by the at least one processor, cause the electronic device to perform the method according to any one of claims 1 to 9.

13. A computer program product, comprising a computer program, wherein the computer program is executed by a processor to implement the method according to any one of claims 1 to 9.

14. A computer-readable storage medium, the computer-readable storage medium having computer-executable instructions stored thereon, wherein the computer-executable instructions are executed by a processor to implement the method according to any one of claims 1 to 9.
